Europäisches Patentamt

(19) European Patent Office

Office européen des brevets

(11) Publication number: **0 177 119**

**A2**

(12) # EUROPEAN PATENT APPLICATION

(21) Application number: **85304664.7**

(22) Date of filing: **01.07.85**

(51) Int. Cl.⁴: **A 61 L 2/24**
**G 01 M 3/32**

(30) Priority: **05.10.84 US 658397**

(43) Date of publication of application:
**09.04.86 Bulletin 86/15**

(84) Designated Contracting States:
**DE FR GB SE**

(71) Applicant: **AMERICAN STERILIZER COMPANY**
**2222 West Grandview Boulevard**
**Erie PA 16512(US)**

(72) Inventor: **Miraldi, Peter Thomas**
**3112 Colonia Avenue**
**Erie, PA 16506(US)**

(72) Inventor: **Kaehler, Kristine Margaret**
**5721 Carriage Hill Drive**
**Erie, PA 16509(US)**

(72) Inventor: **Nevens, Richard Graham**
**2603 Myrtle Street**
**Erie, PA 16508(US)**

(72) Inventor: **McBride, Richard Albert**
**448 East 35th Street**
**Erie, PA 16508(US)**

(74) Representative: **Smith, Norman Ian et al,**
**F.J. CLEVELAND & COMPANY 40-43 Chancery Lane**
**London WC2A 1JQ(GB)**

(54) **Automatic vacuum leak test method.**

(57) A method of automatically testing a steam sterilizer to detect 1 unacceptable air leak rates includes an air removal and system heating step which includes a plurality of pressure/vacuum pulses, a stabilization step, a vacuum hold step and an air admission step. Pressure readings are automatically taken at preset times at the beginning and end of the vacuum hold step. The leak rate is automatically calculated by the pressure change over time in the vacuum hold period. The method of the present invention eliminates the need for human intervention, and thus human error, during the progression and critical pressure readings of the test.

Fig.2.

- 1 -

"AUTOMATIC VACUUM LEAK TEST METHOD"

The present invention relates to an automatic method for testing the vacuum leak rate of a sterilizer.

In steam sterilization processes, saturated steam of a given temperature produces effective bactericidal results. The presence of air or other noncondensable gases in the chamber or entrapped in the interstices of the load in the chamber cause an unsaturated steam environment and therefore, a less effective sterilization cycle. The air insulates the load and may prevent complete steam penetration. Furthermore, air leaks may cause the sterilizer's temperature sensing devices to detect a different temperature than actually exists in the load.

The conventional method of testing a sterilizer for leaks is to draw a vacuum, hold it and manually measure the change in pressure over time to calculate a leak rate. A stop watch is generally used to measure the time. A pressure reading is taken at a time, $t_1$, and again at a time, $t_2$.

- 2 -

The manual testing method lends itself to both equipment and human error. Monitoring a sterilizer over a period of time by different technicians using different watches does not yield long term reproducible results.

There is a need therefore, for a method of testing air leaks in a sterilizer which will provide reproducible results over a period of time and which will not be subject to human error in observing and recording the relevant data. There is a further need for such a method which is automatic and particularly adapted to each sterilizer. Finally, there is a need for such a method which does not depend on extra equipment and which can be easily performed.

The present invention provides an automatic method for testing the air leak rate in a sterilizer which will provide standardized, reproducible results over a period of time and which can be easily operated without extra equipment.

In a sterilizer having a pressure chamber and a control assembly, the control assembly being adapted to be preprogrammed to effect predetermined sterilizer operations in response to predetermined sterilizer conditions, a method for automatically testing the vacuum leak rate of the sterilizer includes the step of preprogramming the control assembly to effect a sequence of sterilizer operations. The sterilizer operations include removing a predetermined quantity of fluid by drawing a vacuum from the chamber and heating the sterilizer, drawing a vacuum in the chamber to a predetermined level for standardizing test starting points, isolating the chamber at the predetermined vacuum level, preferably about fifty millimeters Hg absolute, for a first predetermined period of time sufficient to permit stabilization of the sterilizer conditions, and maintaining the chamber in isolation for a second predetermined period of time is marked by an initial point in time and an end point in time. The sequence of operations further includes detecting and recording the absolute pressure within the chamber at each of the initial and end points in time of the second predetermined period of time, returning the chamber to atmospheric pressure, preferably by the admission of ambient atmospheric air, and calculating the difference between the absolute pressure detected at the initial and end points of time over the second predetermined period of

- 4 -

time to provide the vacuum leak rate of the sterilizer.

The air removal and sterilizer heating operation includes subjecting the chamber to a plurality of cyclic pressure pulses. Each pressure pulse includes a pressurization phase wherein a condensable vapor having trasferable latent heat, preferably steam, is injected into the chamber to elevate the pressure within the chamber, and an evacuation phase wherein the vapor is removed from the chamber to decrease the pressure within the chamber. The plurality of pressure pulses are sufficient to remove the predetermined quantity of fluid from the chamber and to heat the sterilizer to the predetermined normal working temperature.

The vacuum level in the chamber is also preferably detected and recorded upon completion of the drawing operation and the predetermined vacuum level so detected is preferably employed by the control assembly to effect advance of the sequence to the chamber isolating operation.

0177119

- 5 -

The completion of the first predetermined period of time, preferably about five minutes, of the chamber isolating operation is preferably employed by the control assembly to effect advance of the sequence to the chamber isolation maintenance operation. The completion of the second predetermined period of time, preferably about ten minutes, is preferably employed by the control assembly to effect advance of the sequence to the atmospheric pressure return operation.

The present invention can be better understood if reference is made to the drawings in which:

FIG. 1 is a graph showing the stages of the preferred embodiment of the method of the present invention.

FIG. 2 is a schematic diagram showing the control system of a sterilizer which may be used to control the method shown by the graph of FIG. 1; and,

FIG. 3 is a schematic diagram of a sterilizer in which the method graphed in FIG. 1 may be practiced.

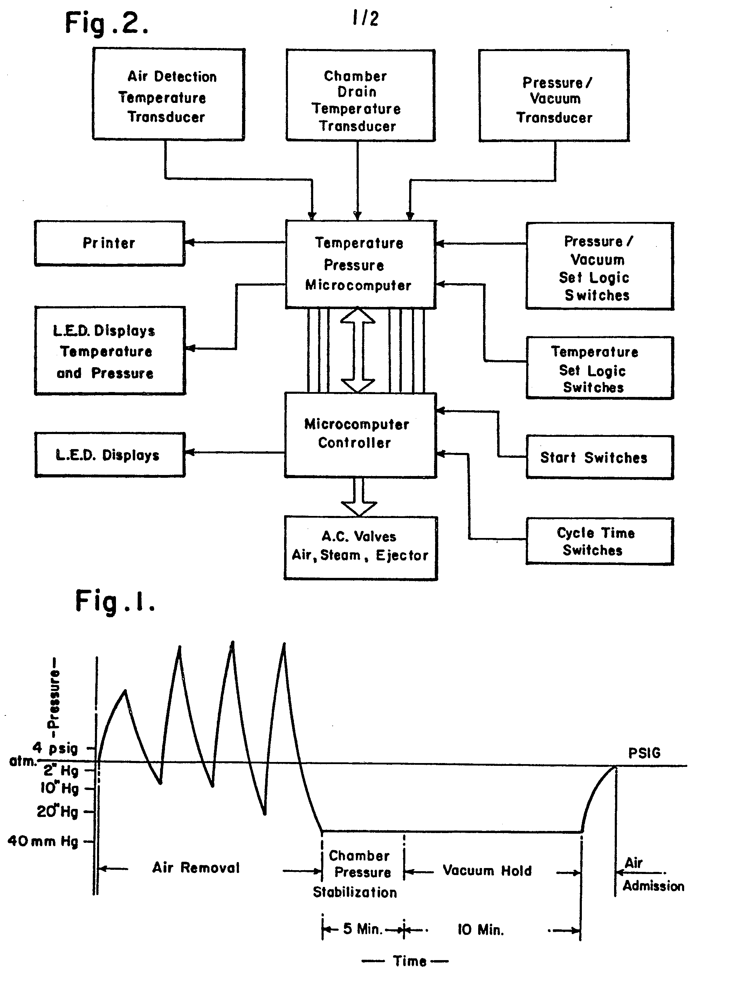

FIG. 1 illustrates the method for testing the vacuum leak rate of a sterilizer provided by the present invention.

- 6 -

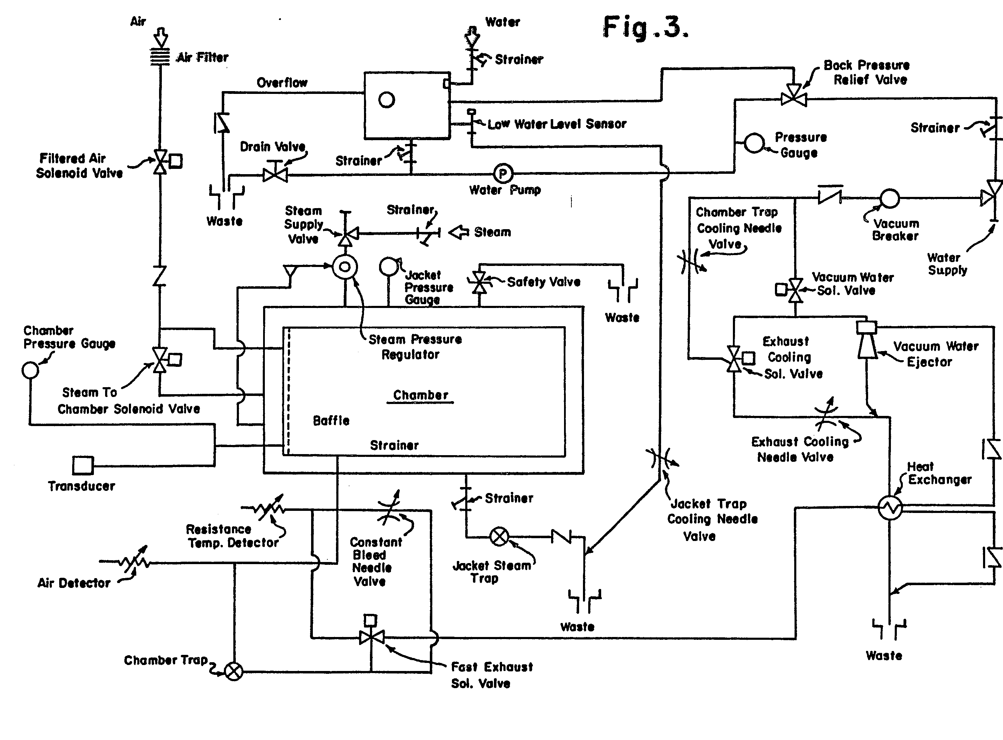

The testing method is adapted for use in a conventional steam sterilizer such as the double walled sterilizer 10 shown in FIG. 3. Sterilizer 10 includes inner walled structure 14 and outer walled structure 18. Chamber 16 is defined within the inner walled structure 14. Steam jacket 20 is defined between outer walled structure 18 and inner walled structure 14.

Sterile filterd air is provided from an air source which passes through air filters 60, solenoid valve 62 and one way check valve 64 to inlet 66. Steam is provided from a source of steam, such as a boiler (not shown), and is delivered through conduit 72 to steam jacket 20. A steam supply valve 56 and a steam pressure regulator 74 control the flow of steam through conduit 72. The steam enters chamber 16 through conduit 68, solenoid valve 70 and inlet 66. A pressure guage 54 and pressure sensing means, or transducer 90 are provided.

The chamber evacuation is achieved by means of the vacuum ejector 84 which is supplied with water by supply valve 104, check valve 106 and vacuum solenoid valve 108. Flow of water through the ejector 84 creates suction in the end of drain 30 and along drain line 44 to evacuate the chamber 16. An optional water source 80 and pump 82 may be provided to accommodate those locations not having readily accessable sources of pressurized water to which to connect the sterilizer 10.

The remainder of the sterilizer and piping schematic of FIG. 3 is shown for completeness but in no way limits the type of sterilizer in which the vacuum leak test of the present invention may be run. Any suitable sterilizer capable of automatically performing the steps of the air leak test described herein below will be able to utilize the present invention.

The steps of the vacuum leak test are preset and automatically controlled by a control assembly which includes the microcomputer, controller and factory set logic switches shown in the diagram of FIG. 2. At least four lines of communication relay information from the microcomputer to the controller, vacuum switch 1, vacuum switch 2, pressure switch 1 and pressure switch 2. A fifth line communicates the information from the controller to the microcomputer that the cycle

- 8 -

has begun. Additional lines for communicating temperature information are also provided.

The logic set switches for pressure, vacuum and temperature are preset, at the factory, according to the levels of pressure, vacuum and temperature which have been determined to be desirable for effecting operation changes in the cycle when the pressure/vacuum sensing means, such as transducer 90, or the temperature sensing means detect levels within the sterilizer. The information is communicated to the microcomputer. When the detected levels match the preset levels of the logic set switches, the microcomputer relays the information by means of the appropriate lines to the controller, which in turn activates the appropriate valve to alter the sterilizer operations. The consequent altered conditions are in turn detected by the appropriate transducers which continue to communicate the information to the microcomputer. L.E.D displays and a printer for recording the conditions and, for purposes of the present invention, the leak test results, are also provided.

- 9 -

The leak test includes the following general steps: the air removal step; the stabilization step; the vacuum hold step; and the air return to atmospheric pressure step. The leak test must be carried out when the sterilizer 10 is at its normal working temperature. In the air removal step, the chamber 16 is therefore subjected to a plurality of cyclic pressure/vacuum pulses which alternately pressurize the chamber for a predetermined period then evacuate the chamber to at least a predetermined minimum vacuum level and for at least a predetermined minimum evacuation time. The precise number of pressure/vacuum pulses and the level of vacuum achieved in each evacuation phase is not critical provided a sufficient amount of air is removed from chamber 16 and the associated piping and chamber 16 and its associated piping are heated to the normal working temperature of the sterilizer. The chamber 16 is pressurized during the pressurization phase of each pulse by a condensable vapor having transferable latent heat, such as steam. The principle of cyclic pressure/vacuum pulses for effectively removing air from and heating a sterilization chamber is described in Young et al. U. S. Patent No. 4,164,538.

- 10 -

In the preferred embodiment, the leak test is started by switching the power on, locking the sterilization chamber door (not shown) and selecting the cycle. The door lock is preferably automatically controlled. The remainder of the leak test is automatically controlled and proceeds without human intervention to the end of the test at step 4.

At the start of the test the chamber is at atmospheric pressure, or 0 psig. Valve 62 is open, and valves 70, 38, 86 and 108 are open. The optional water pump 82 operates throughout the test procedure. Steam is admitted into chamber 16 through inlet 66 for sixty seconds. Then, valve 70 is closed and valve 62 is closed. A vacuum is drawn in the chamber 16 to about ten inches Hg. When the chamber reaches approximately 3-4 psig during the first evacuation pulse, pressure switch 2 drops out, valve 86 is closed, and the vacuum timer begins to run for one minute. When one minute has passed and ten inches Hg has been reached, valves 38 and 108 are closed. Valve 70 is opened to again admit steam to chamber 16 to a level of approximately 26 psig. When that level is achieved, pressure switch 1 picks up, valve 70 closes and valves 38, 86 and 108 open to draw another vacuum. When the pressure falls

- 11 -

to about 3-4 psig pressure switch 2 drops out, valve 86 closes and the vacuum timer again runs for at least one minute until a vacuum of ten inches Hg is achieved, after which valves 38 and 108 close and valve 70 again opens to admit steam into chamber 16 until a pressure of about 26 psig is achieved. The pressure/vacuum pulses continue in that manner with the exception that progressively deeper vacuums are drawn with each proceeding evacuation pulse to a final preset vacuum level of at least 40-50 mm Hg.

When 40 mm Hg is achieved, step 2 proceeds. For a predetermined period of time, about five minutes, all valves are closed and the chamber is isolated. The vacuum drawing means is stopped. The vacuum level at this stage is detected and automatically recorded by the printer. During step 2 the chamber conditions stabilize. The chamber, piping and valve temperatures and the electronic control temperatures, specifically the transducers for sensing conditions, stabilize. Any moisture remaining in the chamber and connecting piping will evaporate, providing an absolute pressure rise. At the end of step 2 the pressure may have stabilized to about 50 mm Hg.

- 12 -

The 40-50 mm Hg level is not critical to the test provided testing always occurs at the same level of vacuum at the end of step 2 to ensure consistency of measurement. However, certain regulatory bodies, specifically, the Department of Health and Social Security in the United Kingdom, do specify vacuum testing levels of at least 50 mm Hg. The specific level preset by the automatic controls may vary according to local regulations and practices. The pressure and time settings can be set to the limits of a particular machine.

At the end of step 2, step 3 begins and the absolute pressure of the chamber 16 is automatically detected, recorded and printed on the sterilizer readout. Step 3, the vacuum hold period proceeds for a predetermined period of time, preferably about ten minutes. All valves remain closed. The pressure sensing means, transducer 90, measures the pressure drift between two points in time at the beginning and end of the ten minute period. The absolute pressure is again automatically recorded on the sterilizer printout.

0177119

- 13 -

At the end of the predetermined period of time in step 3, ambient atmospheric air is admitted into the chamber 16 to return it to atmospheric pressure through valve 62. At the end of step 4, valve 38 is opened.

The leak rate is calculated automatically by the difference in absolute pressure readings taken at the two points in time at the beginning and end of the predetermined ten minute vacuum hold period in step 3, over that predetermined period of time.

Several test leak rate cycles were run. Pressure/vacuum and temperature readings were taken at each point, indicated by numerals 2 through 9 and 11 to 12 on the graph in FIG. 1. The temperature readings are in degrees Celsius and the pressure readings are in mm Hg for the vacuum, or "V" levels and in psig for the pressure, or "P" levels.

TEST 1                 TIME     TEMP.     PRESSURE

Step 1: Air Removal

- 14 -

| | | TIME | TEMP. | PRESSURE |
|---|---|---|---|---|
| Point | 1 | 11:09 | 89.7 | 0.3P |
| | 2 | 11:10 | 129.7 | 23.7P |
| | 3 | 11:11 | 102.1 | 400V |
| | 4 | 11:12 | 130.3 | 26.2P |
| | 5 | 11:13 | 90.8 | 125V |
| | 6 | 11:13 | 130.0 | 26.1P |
| | 7 | 11:14 | 88.1 | 91V |
| | 8 | 11:15 | 129.7 | 26.2P |

Step 2: Chamber stabilization

| | 9 | 11:17 | 98.0 | 34V |
|---|---|---|---|---|

Step 3: Vacuum Hold

| 11 | 11:22 | 86.6 | 48V |
|---|---|---|---|
| 12 | 11:32 | 71.2 | 54V |

Step 4: Air admission

Leak rate = 0.6 mm Hg/min. - acceptable

| Test 2 | TIME | TEMP. | PRESSURE |
|---|---|---|---|

- 15 -

Step 1: Air removal

| | | | |
|---|---|---|---|
| 1 | 12:16 | 88.8 | 0.6P |
| 2 | 12:17 | 130.2 | 23.9P |
| 3 | 12:19 | 102.3 | 400V |
| 4 | 12:19 | 131.5 | 26.1P |
| 5 | 12:20 | 92.5 | 128V |
| 6 | 12:21 | 129.9 | 26.2P |
| 7 | 12:22 | 90.0 | 91V |
| 8 | 12:22 | 130.0 | 26.2P |

Step 2: Chamber stabilization

| | | | |
|---|---|---|---|
| 9 | 12:24 | 98.2 | 34V |

Step 3: Vacuum hold

| | | | |
|---|---|---|---|
| 11 | 12:29 | 85.5 | 56V |
| 12 | 12:39 | 71.2 | 69V |

Step 4: Air admission

Leak rate = 1.3 Hg/min. - unacceptable

- 16 -

Unaccepatable air leak rates have been determined to be any rate greater than 1 mm Hg/min. Accordingly, the first test would indicate an acceptable rate and the second test an unacceptable rate.

- 17 -

CLAIMS

1. In a sterilizer having a pressure chamber and a control assembly, said control being adapted to be preprogrammed to effect predetermined sterilizer operations in response to predetermined sterilizer conditions, a method for automatically testing the vacuum leak rate of said sterilizer comprising:

preprogramming said control assembly to effect a sequence of sterliizer operations, said sterilizer operations comprising removing a predetermined quantity of fluid by drawing a vacuum from said chamber and heating said sterilizer to a predetermined normal working temperature of said sterilizer;

drawing a vacuum in said chamber to a predetermined level for standardizing test starting points;

isolating said chamber at said predetermined vacuum level for a first predetermined period of time sufficient to permit stabilization of sterilizer conditions;

maintaining said chamber in isolation for a second predetermined period of time, said second predetermined period of time being marked by an initial point in time and an end point in time and

- 18 -

detecting and recording the absolute pressure within said chamber at each said initial and end points in time;

returning said chamber to atmospheric pressure; and

calculating the difference between the absolute pressures detected at said intial and end points in time over said second predetermined period of time to provide said vacuum leak rate for said sterilizer.

2. A method as recited in claim 1 wherein said chamber air removal and sterilizer heating operation comprises subjecting said chamber to a plurality of cyclic pressure pulses, each said pressure pulse including a pressurization phase wherein a condensable vapor having transferable latent heat is injected into said chamber to elevate the pressure within said chamber and an evacuation phase wherein said vapor is removed from said chamber to decrease the pressure within said chamber, said plural of cyclic pressure pulses being sufficient to remove said predetermined quantity of fluid from said chamber and to heat said sterilizer to said predetermined normal working temperature.

3. A method as recited in claim 2 wherein said

- 19 -

condensable vapor is steam.

4. A method as recited in claim 1 wherein said predetermined vacuum level is at least about fifty millimeters Hg absolute.

5. A method as recited in claim 1 wherein said first predetermined period of time is about five minutes and said second predetermined period of time is about ten minutes.

6. A method as recited in claim 1 wherein said chamber is returned to atmospheric pressure by admitting ambient atmospheric air to said chamber.

7. A method as recited in claim 1 wherein the vacuum level in said chamber is detected and recorded upon completion of said vacuum drawing operation and said predetermined vacuum level so detected is employed by said control assembly to effect advance of said sequence to said chamber isolating operation.

8. A method as recited in claim 1 wherein the completion of said first predetermined period of time of said chamber isolating operation is employed by said control assembly to effect advance of said sequence to said chamber isolation maintenance operation and the completion of said second predetermined period of time is employed by said control assembly to effect advance of said sequence to said atmospheric pressure return operation.

# Fig.2.

| Air Detection Temperature Transducer | Chamber Drain Temperature Transducer | Pressure/ Vacuum Transducer |
|---|---|---|

| Printer | | Temperature Pressure Microcomputer | | Pressure/ Vacuum Set Logic Switches |
|---|---|---|---|---|

| L.E.D. Displays Temperature and Pressure | | | | Temperature Set Logic Switches |

| L.E.D. Displays | | Microcomputer Controller | | Start Switches |

| | | A.C. Valves Air, Steam, Ejector | | Cycle Time Switches |

# Fig.1.

— Pressure —

4 psig
atm.
2" Hg
10" Hg
20" Hg
40 mm Hg

PSIG

Air Removal | Chamber Pressure Stabilization | Vacuum Hold | Air Admission

— 5 Min. — | 10 Min.

— Time —

Fig.3.